# EUROPEAN PATENT APPLICATION

(11) **EP 3 785 823 A1**
(43) Date of publication of application: **03.03.2021**
(21) Application number: 19194693.8
(22) Date of filing: 30.08.2019
(51) Int. Cl.: B22F 1/00, C07K 14/47, C08L 89/00, C22C 32/00, C08K 3/08

(54) **LIGHT GOLD**

(71) Applicant: ETH Zurich, 8092 Zürich (CH)
(72) Inventor: van 't Hag, Leonie, 7609 WC Almelo (NL); Mezzenga, Raffaele, 8604 Volketswil (CH)
(74) Representative: Grimm, Siegfried

(57) **Abstract**

The present invention relates to novel composite materials comprising elemental gold in the form of single crystals, amyloid fibrils and a polymer. This composite material is similar to glassy plastics yet lighter than aluminum and has a golden shining similar to 18K gold. Due to its unique properties, this composite is termed "light gold". This composite material suits, for example, watches, jewelry, radiation shielding, catalysis and electronics. The invention further provides for environmentally friendly methods to manufacture such composite materials.

## Description

The present invention relates to novel composite materials comprising elemental gold, amyloid fibrils and a polymer. This composite material is similar to glassy plastics yet lighter than aluminum and has a golden shining similar to 18K gold. Due to its unique properties, this composite is termed "light gold". This composite material suits watches, jewelry, radiation shielding, catalysis and electronics. The invention further provides for environmentally friendly methods to manufacture such composite materials.

It is known that gold has many industrial and commercial applications. First, Gold stimulates an ever-lasting craze not only in jewelry and decoration markets. Second, due to its combination of exceptional physical and chemical properties, gold also attracts great interests in many different fields of science and technology, including applications in catalysts, sensors and optoelectronic devices.

EP1918047 discloses composite materials comprising Carbonate ester and gold particles of less than 0.5 micron. These composite materials are obtained by co-extruding the polymer and the particles. This process, although suitable in principle, proved to be non-suitable for commercial applications. Due to its agglomeration properties, it is not possible to use gold in the form of single crystals in this process. As a consequence, the materials described in that document are inferior in view of physical and visual properties.

WO2014/124546 describe self-assembled protein-gold hybrid materials in solution and self - supported thin films comprising these hybrid materials. The document fails in teaching how to combine such hybrid materials with polymers.

Hence, it is the object of the present invention to provide improved composite materials and to provide improved methods for manufacturing such materials.

These objectives are achieved by the composite material as defined in claim 1 and the manufacturing method as defined in claim 7. Further aspects of the invention are disclosed in the specification and independent claims, preferred embodiments are disclosed in the specification and the dependent claims.

The present invention will be described in more detail below. It is understood that the various embodiments, preferences and ranges as provided / disclosed in this specification may be combined at will. Further, depending on the specific embodiment, selected definitions, embodiments or ranges may not apply.

As used herein, the terms "a", "an,", "the" and similar terms used in the context of the present invention (especially in the context of the claims) are to be construed to cover both the singular and plural unless otherwise indicated herein or clearly contradicted by the context. The term "containing" shall cover "comprising", "essentially consisting of" and "consisting of"

The present invention will be better understood by reference to the **figures**.

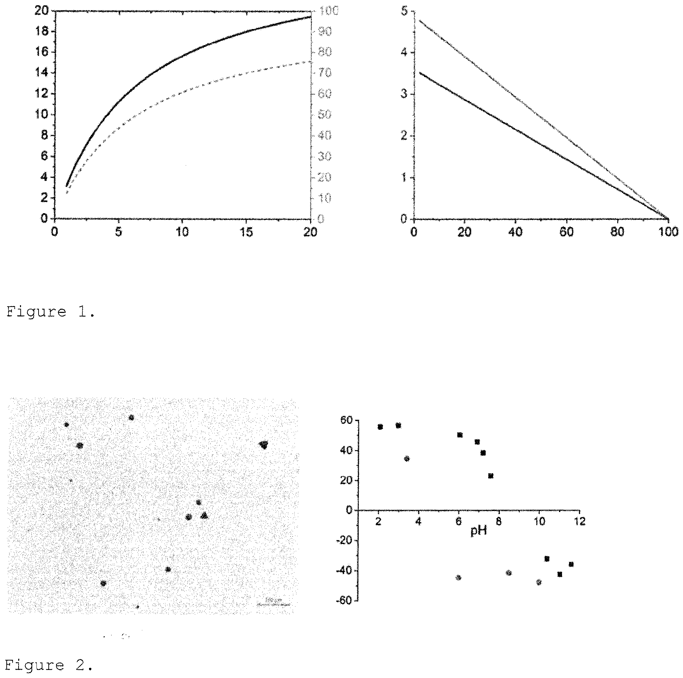

Figure 1 (Left graph): Gold alloy maximum density (left y-axis continuous line, g/cm3) and gold volume fraction (right y-axis(dotted line), % v/v) as a function of the density of the additive in an 18 karat or 75% w/w gold (*ρ*Au= 19.3 g/cm3) alloy. Polystyrene (PS) as used herein has a density of 1.04 g/cm3, leading to a maximum density of 3.6 g/cm3 and 14% v/v gold. BLG is an additional component which has a density of 1.50 g/cm3 leading to a maximum density of 4.9 g/cm3.
Figure 1 (Right graph): The apparent density of these materials (g/cm3) as a function of the porosity (Φ, in percent) is shown for 18 karat gold with PS (lower line) or BLG (upper line) as the additive.
Figure 2 (Left picture): Bright-field microscopy of gold platelets at 2.6 mM HAuCl4, the scale bar is 100 µm. Figure 2 (Right graph): Zeta-potential (mV) of Au crystal dispersions with BLG fibers (solid circles) and PS-NH2 (solid squares) with a diameter of 520nm at pH 2-12.

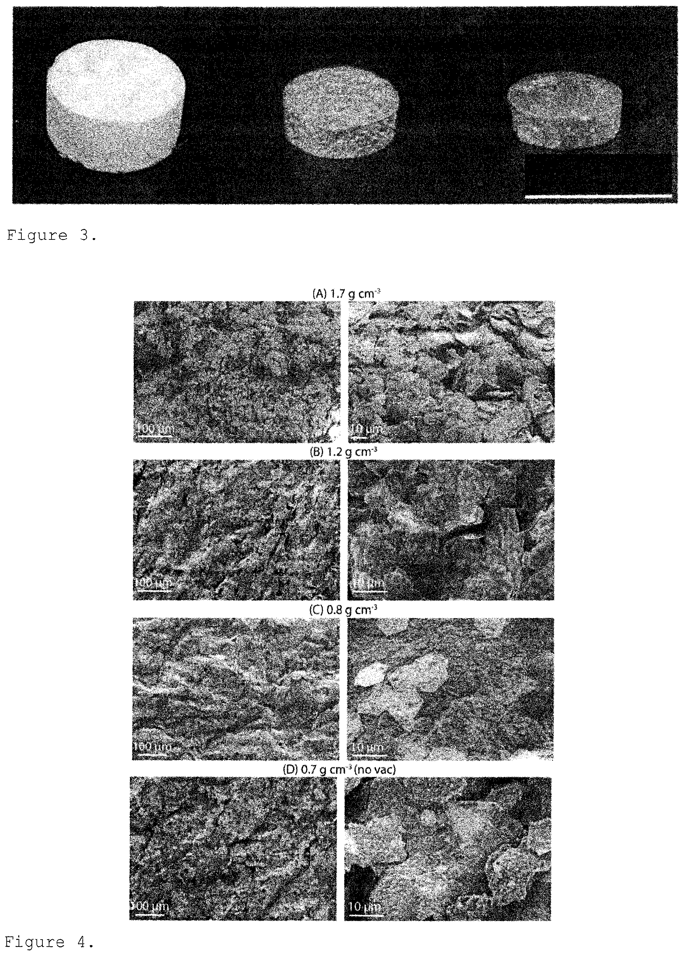

Figure 3. Photographs of the gold crystal, amyloid and polystyrene (Au-PS) hybrid aerogel with a final density of 1.7 g/cm3 upon processing. Sample of 170 mg: (Left) after supercritical CO2 drying of the hydrogel and (Center) after annealing of the polystyrene. (Right) Upon polishing with super fine P1200 sandpaper with 15.3 µm average particle diameter which reduced the weight to 150mg. Scale bar 1cm.
Figure 4: Scanning electron microscopy (SEM) of the inventive composite materials. (top 3 rows) were annealed under a vacuum of 30mbar, while (4^{th} row) was annealed under atmospheric pressure. The final density of each of the samples is indicated in the individual captions. Scale bars of 100µm (left column) and 10µm (right column) are shown.

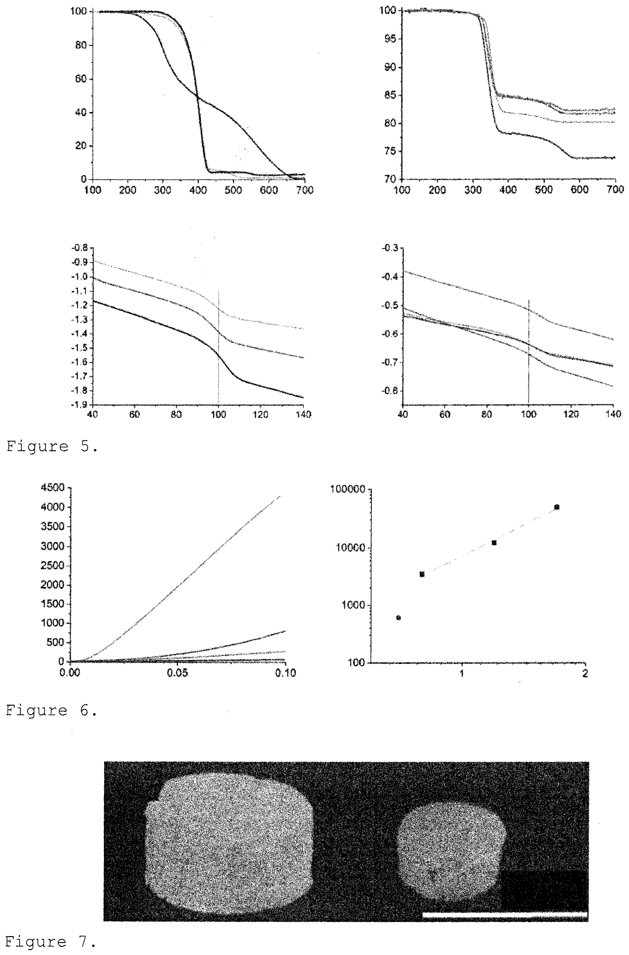

Figure 5: (top row) Thermal gravimetric analysis (TGA; mass (%) vs. Temperature °C) and (second row, Heat flow (mW vs. Temperature °C) dynamic scanning calorimetry (DSC) measurements of the hybrid Au-PS materials as shown in Figure 4. In (left panel, top row) the BLG sample was prepared by freeze-drying BLG fibers and is the bottom curve at 300°C. Furthermore, annealed PS-NH2 is the middle curve at this temperature. PS-NH2 - BLG annealed is the top curve at 300°C and is the sample shown in Figure 7 (#7). In (right panel,top row) the curves show results for samples with 1.2, 1.7, 0.8 and 0.7 g/cm3 from bottom to top at 600 - 700 °C. A vertical line at 100°C is shown in the DSC plots in (low row) as a guide for the eye for the glass transition temperature of PS. DSC curves shown are the cooling curves following a heating curve up to 160°C. In (left panel, bottom row) PS-NH₂ - BLG annealed, PS-NH₂ powder and PS-NH₂ annealed are shown from bottom to top. In (right panel, bottom row) results for samples with 1.2, 1.7, 0.8 and 0.7 g/cm³ are shown from bottom to top at 40°C.
Figure 6. Mechanical properties of the inventive composite materials. Left panel: Compressive stress strain curves for materials annealed under vacuum (0.8 - 1.7 g/cm3) and without vacuum (0.7 g/cm3, no vac, bottom curve). The curves refer to densities of 0.7, 0.8, 1.2, 1.7 g/cm3, from bottom to top curves). Right Panel: Young's moduli extracted from the slope from 0.05 - 0.1 strain. The linear fit was used to obtain the scaling behaviour and is shown through data for samples that were annealed under vacuum.
Figure 7. Photographs of an amyloid and polystyrene (Au-PS) hybrid aerogel sample #7. Sample of 78 mg: (A) after supercritical CO₂ drying of the hydrogel and (B) after annealing of the polystyrene: *ρ*ₐₚₚ = 1.2*g*/*cm*³ and 0% porosity.

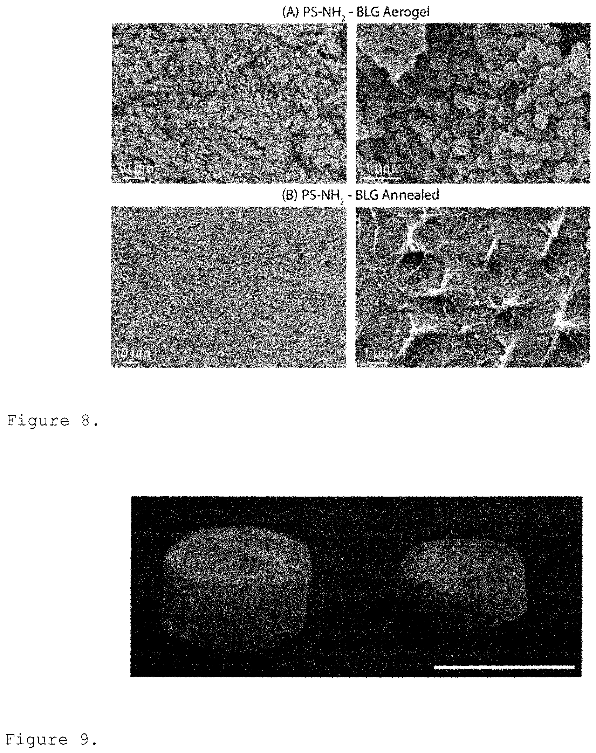

Figure 8. Scanning electron microscopy (SEM) of the polystyrene and amyloid hybrid materials. (A) Shows PSNH₂ - BLG sample #6 before annealing and (B) shows PS-NH₂ - BLG sample #7 after annealed under a vacuum of 30 mbar.
Figure 9. Photographs of the 15 karat purple hybrid aerogel sample #5 consisting of Au crystals and nanoparticles, BLG fibrils and PS-NH₂. Sample of 48 mg: (Left) after supercritical CO2 drying of the hydrogel and (Right) after annealing of the polystyrene: *ρ*app = 0.4 *g*/*cm*³ and with *ρ*max= 2.2 *g cm*-3 gives a porosity of 82%. Scale Bar: 1cm.

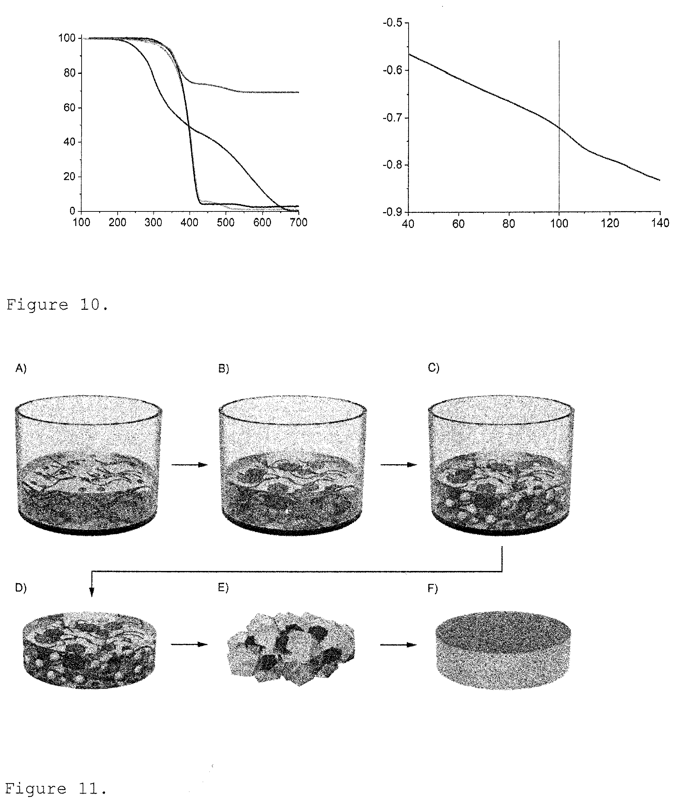

Figure 10, (Left) TGA: mass (%) vs. Temp. (°C); shown together with reference data as shown in Figure 5. From bottom to top at 600°C the following curves are shown: PS-NH2 annealed, PS-NH₂ - BLG annealed, BLG, and example #5 (15 karat, 69 % - 3 % gold).
   Right: DSC measurements (heat (mW) vs. Temp. (°C)) of the hybrid Au-PS materials (example #5) as shown in Figure 9. A vertical line at 100°C is shown in the DSC plots as a guide for the eye for the glass transition temperature of PS. DSC curve shown is the cooling curves following a heating curve up to 160°C.
Figure 11: Illustration of the Light Gold production process, wherein D) shows the Hydrogel, E) the Aerogel and E) the inventive composite material. The manufacturing process is described in more detail in the second aspect of the invention, a brief summary thereof is as follows.
   Step (a), shown in figs. 11A and 11B: A mixture of amyloid fibrils (e.g. BLG fibrils) and gold ions are mixed (A). They form gold single crystals upon incubation (e.g. at 60°C for 16 hours; (B)).
   Step (b), shown in figs. 11C and 11D: A colloidal polymer latex (e.g. polystyrene latex) is added to obtain a combined composition (C); Hydrogel formation occurs upon increase of the ionic strength (e.g. the diffusion of NaCl through a membrane; (D)).
   Step (c), shown in fig. 11E: Drying (e.g. Supercritical CO₂ drying) results in the formation of a robust gold aerogel (E).
   Step (d), shown in fig. 11F: Annealing of the aerogel above the glass transition temperature of the polymer (e.g. of 100°C in case of PS) results in the inventive composite material (F).

Further details on the figures are provided in the experiments below.

In a **first aspect**, the present invention relates to composite materials ("light gold", "composites") containing (i.e. comprising or consisting of) amyloid fibrils, elemental gold and a polymer; whereby said elemental gold is present as single crystal gold platelets and homogeneously distributed within the polymer and whereby said composite has a density of 0.7 - 3.9 g/cm³. It is similar to a glassy plastics yet lighter than aluminum and suits watches, jewelry, radiation shielding, catalysis and electronics. The density and stiffness, as well as the color, of the material can be tuned depending on what is desired for the application. This aspect of the invention shall be explained in further detail below:
**Amyloid Fibrils:** The term "amyloid fibrils" is generally known in the field and particularly describes fibrils made by proteins or peptides prevalently found in beta-sheet secondary structure. Accordingly, the term amyloid fibrils excludes native proteins.

Without being bound to theory, the roles played by amyloid fibrils are believed to be multiple: they allow reduction of gold salts into platelets, their colloidal stabilization, and gel formation.

Advantageously, the amyloid fibrils have high aspect ratio, preferably with ≤ 10 nm in diameter and ≥ 1µm in length. Advantageously, the amyloid fibrils have a highly charged surface. The term highly charged surfaces is generally known in the field and particularly describes surfaces showing electrophoretic mobilities of the order 2 µm 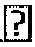 cm/V 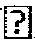 s at pH 4.

**Elemental Gold:** The inventive composite materials comprise gold in elemental form, i.e. oxidation state +/-0. The elemental gold may be present in various forms, such as gold platelets, nanoparticles and combinations thereof. Gold Platelets: Advantageously, the elemental gold is present in the form of gold platelets, preferably single crystal gold platelets. Such platelets have a high aspect ratio, such as 500: 1, preferably 800:1; typical sizes are 5- 20 pm, preferably 10 - 20 µm; and the thickness only of 100 nm or less, such as 25 nm or less. Without being bound to theory, it is believed that the high aspect ratio gold single crystals provide metal conductivity, and golden shining. The resulting materials have well-defined layered hierarchical structures and combine physical properties from both individual constituents, such as, for example, water-responsive and tunable conductivities from insulating to metallic levels.

Nanoparticles: In an alternative embodiment, the elemental gold is present in the form of nanoparticles preferably crystalline nanoparticles ("Nanocrystals"). Nanoparticles distinguish from platelets by its approximately isometric shape, i.e. aspect ratio below 10:1, preferably below 2:1. Typical sizes of nanoparticles are in the range of 10 - 1000 nm, e.g. 20 - 100 nm. Nanoparticles, may be beneficial for applications where the material's golden appearance is of less relevance.

Combination of platelets and nanoparticles: The above materials may also be simultaneously present in the inventive composite materials.

The amount of elemental gold may vary over a broad range, depending on the intended use of the inventive composites. Typically, elemental gold amounts to 10-99 wt.%, preferably 30 - 99 wt.% of the total weight of the composite material. Accordingly, composite materials, of the present invention may have a gold content of 9ct, 14ct, 18ct 21ct, 21.6ct, or 22ct, for example.

**Hybrid Materials:** The above mentioned elemental gold is stabilized via amyloid fibrils ("amyloid fibrils, Gold crystals"). The term "hybrid material" refers to materials comprising both, organic components as well as inorganic components in intimate contact. Such hybrid material may be present as a dispersed phase in an aqueous suspension (this is typically the case during manufacturing)as well as in the hydrogels, aerogels and inventive composite materials described herein.

These hybrid materials are made of 2-dimensional and 1-dimensional nanoscale building blocks; elemental gold (particularly single crystal gold nanoplatelets typically form 2D- building blocks), and amyloid fibrils typically form 1D-building blocks. The structure of these hybrid materials is complex, and may be described as homogenous in 3 dimensions, including regions randomly distributed in 3 dimensions and regions of layered structures. Such material being described e.g. in WO2014/124546.

The size of the hybrid material may vary; typically a range of 20 - 1000 nm is found. Without being bound to theory, it is believed this particle size contributes to its stability in aqueous dispersions, making it suitable for the applications outlined herein.

**Polymer:** A wide range of polymers may be used. Suitable are, for example polymers selected from the group of polyolefines (including polyethylenes and polypropylenes (PE and PP)); polyacrylates (including Polymethylmethacrylates (PMMA)) and polystyrenes (PS); preferably polystyrenes.

Advantageously, the polymer is obtained from a latex (i.e. polymer dispersion in an aqueous medium) with a diameter below 10 µm, preferably below 5000 nm, such as 300 - 500 nm.

In the inventive composite materials, the polymer forms a matrix wherein the elemental gold is homogeneously distributed.

**Composite material:** In addition to the chemical composition, the inventive composite material may be characterized by physical parameters.

Advantageously, the composite material has a density in the range of, or lower than, aluminium Suitable ranges include 0.7 -3.9 g/cm3, preferably 1.5-3.9 g/cm3, particularly preferably 2.5 - 3.5 g/cm3.

Advantageously, the composite material has a porosity of less than 80%, such as 60 - 80 %.

Advantageously, the composite material has a glass transition temperature in the range of 80 - 120°C, such as 105 °C.

Advantageously, the composite material has a golden shining, not to be distinguished from pure gold by the naked eye.

Advantageously, the composite material has a Young's modulus in the range of 10 MPa to 30'000 MPa, preferably 50 MPa to 1000 MPa.

**Product-by-process:** In a further embodiment, the invention also provides for a composite material obtainable by the method described herein, or obtained according to the method as described herein. In this embodiment, the amyloid fibrils are preferably prepared from food-grade proteins; preferably selected from the group consisting of *β-*lactoglobulin, lysozyme, ovalbumin, and serum albumines. It is considered particularly advantageous that broadly available, inexpensive food-grade proteins are suitable starting materials for manufacturing the inventive composites. Further, in this embodiment the single crystal gold platelets are simply prepared by reducing gold salts in an aqueous solution optionally further stabilized with *β*-lactoglobulin amyloid fibrils in colloidal state. Further, in this embodiment, the polymer latex is an aqueous polystyrene dispersion. It is considered particularly advantageous using such green chemistry for manufacturing light gold.

In a **second aspect**, the invention relates to a method of manufacturing the inventive composite materials. Briefly, a hydrogel is prepared from a polymer latex and amyloid fibrils-Gold crystals; this hydrogel is converted into an aerogel followed by annealing to thereby obtain the inventive composite material. The inventive composite material shows a homogeneous microstructure in which the shining gold single crystal platelets are embedded in the polymer matrix. The inventive composite materials, obtainable by the method described herein, show remarkable properties that none of the constituents could generate alone. This aspect of the invention shall be explained in further detail below:
Advantageously, the inventive method comprises the steps of:
(a) providing a first aqueous composition comprising amyloid fibrils-Gold crystals and a second aqueous composition comprising a polymer latex; and
(b) combining said first and second composition (step b1), followed by controlled increase of ionic strength (step b2) to thereby obtain an organic-inorganic hydrogel; and
(c) converting the thus obtained hydrogel into an aerogel by removing the solvent; and then
(d) annealing the thus obtained aerogel at elevated temperatures, optionally at reduced pressure, to thereby obtain the composite material.

The individual process steps are known per se, but not yet applied to the specific starting materials and visualized in fig.11. The obtained composite material may be further processed according to methods well established (c.f. step (e) below).

It is considered particularly advantageous that the entire manufacturing is green and eco-friendly.

It is considered particularly advantageous that important properties of the inventive composite material, including density, stiffness and color, may be tuned in s simple way by adjusting the individual process steps. For example, the final apparent density and porosity of the inventive composite material was found to be determined by the volumetric concentration in the starting solution used for hydrogel formation.

It is considered particularly advantageous that the method provides composite materials with well-organized structure and unprecedented properties.

It is further considered particularly advantageous that the obtained materials have unique optical properties, such as fluorescent and optic-grade golden color.

### Step (a), Starting Materials

The inventive method involves two main starting materials, hereinafter first aqueous composition and a second aqueous composition. The first aqueous composition comprises amyloid fibrils-Gold crystals, the second aqueous composition comprises a polymer latex. These starting materials are known per se.

At present, the first composition is not a commercial item and may be obtained according to steps (a1)-(a3). Briefly, The first composition may be obtained by (a1) Growing protein amyloid fibrils, preferably from β-lactoglobulin or lysozyme; (a2) Growing single crystal platelets, preferably from chloroauric acid, in the presence of amyloid fibrils; (a3) optionally concentrating the thus obtained amyloid fibrils-single crystal gold platelets in suspensions.

The second composition is commercially available and discussed in step (a4).

**Step a1:** The synthesis of amyloid fibrils is a known technology. Suitable is in particular protein hydrolysis followed by β-sheets driven fibrillation, as described e.g. in Jung et al. (Biomacromolecules. 2008, 9, 2477-2486). Suitable starting materials are food-grade proteins, which are structural stable, wide accessible and inexpensive. Such starting materials allow preparation of amyloid fibrils, such as *β*-lactoglobulin. Suitable proteins may be selected from the group consisting of *β*-lactoglobulin, lysozyme, ovalbumin, and serum albumines.

The self-assembly process is facile and controllable. Typical process parameters include incubating protein solution (e.g. 2 wt.% *β*-lactoglobulin) for a prolonged, period of time (e.g. 6 h) under acidic conditions (e.g. pH ∼ 2), low ionic strength (e.g. I ≤ 20 mM), high temperature (e.g. T ∼ 90 °C).

BLG amyloid fibrils are rod-like structures with a diameter of ∼5 nm and a contour length spanning several micrometers.

**Step a2:** The synthesis of single crystal gold platelets is a known technology. Suitable is in particular the green chemistry method of Bolisetty et al. (Journal of Colloid and Interface Science. 2011, 361, 90-96; WO2014/124546) that involves reducing an aqueous solution of gold salts which is stabilized with amyloid fibrils in colloidal state. This method provides for single crystal gold platelets with super large size (eg. 10 - 20 µm) and high aspect ratio (up to 10³). Under controlled conditions (particularly pH, temperature, amyloid fibril concentration), amyloid fibrils can act both as a reducing agent and a stabilization agent to synthesize the gold platelets and to provide high colloidal stability. In other terms, due to the three-fold roles played by the protein fibrils, only two materials were involved in the fabrication of these unique gold. Typical process parameters include mixing 0.67 wt. % amyloid fibrils of step a1) and 0.066 wt.% chloroauric acid, and then incubating at pH 2 at elevated temperatures (e.g. 60 °C) for a prolonged period of time (e.g. 16 h).

As discussed above, BLG further acts as a reducing agent to form gold single-crystals of 10 - 20 µm at 2.6 mM HAuCl4 herein, Figure 2(A). The zeta potential of this solution at pH 3 - 10 is shown in Figure 2(B). Below the isoelectric-point of BLG (pH at which it carries no net charge: pI = 5)35 the dispersion showed a positive zeta-potential and above the pI it showed a strong negative zeta-potential. This shows that the colloidal solution is stable and that the particles are positively charged at low pH and negatively charged above pH = 5. Metallic gold is not charged and for this reason the charge of the gold crystal-BLG dispersion followed the isoelectric point of BLG.

The synthesis of nanocrystalline gold is a known technology. Suitable is, for example reduction of a gold salt in the presence of NaBH4. The resulting nanoparticles are of 26 nm in diameter and give a purple color of the inventive composite material. In this embodiment, amyloid fibrils act as a stabilization agent to provide high colloidal stability.

**Step a3:** Non-reacted starting materials may be separated, thereby concentrating the inventive composites in suspension. This may be done by simple centrifugation, and discharging / recycling the non-reacted supernantant aqueous amyloid phase.

**Step a4:** The preparation of an aqueous polymer latex is a known technology; such latex (i.e. polymer dispersion in an aqueous) are commercial items. Suitable polymers are discussed above, PE, PP, PMMA and PS, particularly PS, are suitable. Polymer dispersions may contain further additives, such as surface-active compounds (tensides, protective colloids). Particle sizes of the polymer may vary over a broad range, typically within 10 nm to 10 micrometers. Particle concentration may vary over a broad range; typically within 5 - 75 w/v%, such as 10 - 60 w/v%, e.g. 50 w/v%. Latex parameters may be adjusted according to the specific manufacturing process by conventional means.

Specifically, the polystyrene latex (PS-NH2 Ø 520 nm) that was described in the examples showed good stability (zeta potential ± 40) and an isoelectric point around pH = 9.

### Step (b) formation of organic-inorganic hydrogel

Converting a polymer latex into a hydrogel is a process known per se and may be applied to the present starting materials. Hydrogel formation typically involves combination of the starting materials (step b1) and effecting gelation (step b2)

Step b1: In an embodiment, the first and second composition are combined at pH 7, where the polymer latex was positively charged and first composition was negatively charged. In an alternative embodiment, the first and second composition are combined at pH 2-3 where there is electrostatic repulsion between all colloids. This embodiment provides more control over the sample morphology and to obtain a homogeneous material on the microscale. Step b2: Diffusion of salt through a membrane then resulted in charge screening and controlled hydrogel formation. Advantageously, BLG fibril concentration in the solutions was 0.5 - 2% w/v, which is the range in which they can form a gel as shown in the phase diagram. The ionic strength may be controlled by contacting the combined compositions with a saline solution via a diaphragm.

### Step (c) formation of organic-inorganic aerogel

Converting a hydrogel into an aerogel is a process known per se and may be applied to the present hydrogel.

In one embodiment, the water of the hydrogel is replaced by a low boiling organic solvent, such as ethanol, prior to conversion to an aerogel.

In one embodiment, scCO2 is used for aerogel formation (c.f. Fig.3A where the polystyrene nanoparticles significantly scatter light giving it a white appearance). According to the prior art, Supercritical CO2 drying of polystyrene results in foaming depending on the molecular weight of the material; further, Supercritical CO2 was suggested to plasticize the matrix and lower the apparent Tg to ambient temperatures. Against this adverse effects reported, the present inventors found the conversion into an aerogel proceeds smoothly with the present starting material. Without being bound to theory, it is speculated that the significantly larger molecular weight and the volume of each particle contribute to the favorable process. It was further found that freeze drying, although suitable for aerogel formation in general, results in unfavorable ice templating making it less suitable in the present case.

### Step (d) formation of inventive composite material

Annealing may take place at elevated temperatures, such as 100-250°C, preferably 150-200°C. Annealing times may vary over a broad range and depend on the size of the inventive composite material, typically 1 min - 1 day, such as 8 hours.

Annealing may take place at reduced pressure and / or in the presence of a protecting gas.

After annealing (Figure 3(B)) and polishing of the surface (Figure 3(C)) the shining gold color is visible. The aerogel volume reduced by a factor four during annealing, resulting in a final apparent density of 1.7 g/cm3. The maximum density based on the composition of 76% w/w Au (15% v/v), 20% w/w PS-NH2 (75% v/v) and 4% w/w (10% v/v, 1.5 g/cm3) BLG was calculated to be 3.9 g/cm3. That results in a calculated porosity (Φ) of 57% v/v.

### Step (e) Additional steps

The inventive process may be accomplished by further steps, e.g. preceding step (a) or following step (d), including purification, further processing, assembling and other process steps known to the skilled person.Advantageously, the inventive method comprises one or more finishing steps (e), including polishing the obtained composite material (e1), casting / extruding the obtained composite material (e2), and coating the obtained composite material on a substrate (e3).

**Step (e1), polishing.** As the inventive composite material comprises Au homogeneously distributed within a matrix of polymer, known polishing technologies may be applied to thereby improve its appearance.

**Step (e2), casting:** Due to its properties of a polymer, common casting and extruding technologies may be applied.

**Step (e3), coating:** To obtain coated substrates, printing and coating technologies may be used. Accordingly, the invention provides for a method, comprising the step of printing a suspension comprising the hybrid composites as described herein. Suitable printing techniques include ink jet printing or micro-contact printing.

The intermediate materials described herein, particularly hydrogels and aerogels, are also subject of the present invention. Further, the invention also relates to the use of a polymer latex for manufacturing the inventive composite material. Further, the invention also relates to the use of hybrid materials described herein (amyloid fibrils in intimate contact with Gold crystals) for manufacturing the inventive composite material.

In a **third aspect**, the invention also relates to various uses of the inventive composite materials and to articles comprising or consisting of a composite material as described herein.

As outlined above, traditional 18 karat gold alloys with other metals typically result in a final density of ~15 g/cm3. Lighter gold blends such as foams and aerogels, typically lead to poor/unacceptable mechanical properties, making them unsuitable for large scope applications. A light gold, as described herein, with density 5 to 10 times lighter than typical blends (density similar or lower than aluminum: ∼3 g/cm3) is described herein. This new composite material has mechanical properties comparable or superior to glassy plastics. It can significantly enhance the wearer experience of watches and jewelry. Further, such composite material also improves transport and material properties for radiation shielding applications (e.g. in space), as well as for catalysis and for electronics. As such, the light gold described herein fills a niche which is currently unoccupied in the realm of industrially relevant gold blends. It may replace gold alloys in present applications and open the way to unexplored applications. The inventive composite material may thus be present in the form of a shaped article, a self-supporting film; or a coating on a substrate.

Accordingly, the invention provides for an article, selected from the group consisting of decorative articles, which are partly or fully coated with the composite described herein or which are printed with an ink comprising the composite described herein; ornamental articles comprising or consisting of a composite material described herein; electrical devices, comprising a composite material described herein; catalytic material, either in the form of monolith or in the form of granules / pellets containing the inventive composite material.

In embodiments of the invention, the inventive composite material is present as a shaped article, such as a semifinished product. This is typically the case once manufacturing is completed. In this form, the inventive composites have a golden appearance and handling properties of a thermoplastic polymer. The shaped article may be an ornamental article or part of an ornamental article. Ornamental articles include jewelry and watches.

In a further embodiment of the invention, the inventive composite is present as a coating on a substrate. A broad range of substrates may be coated, depending on the intended use of the inventive composite. The coating may be the top coating, thereby replacing traditional leaf gilding of articles. The coating may also be a functional layer, e.g. in a sensor or electrical device. Accordingly, the invention also provides for an article comprising a substrate and a coating, said coating consisting of an inventive composite material as described herein. Such articles include decorative articles, such as packaging materials, decorative articles which are partly or fully coated with the inventive composite or which are printed with an ink comprising the inventive composite. Such articles further include electrical devices comprising wires, microdevices or electrical conductors made of the inventive composites. Such articles further include sensors comprising the inventive composites as functional layer or functional element, particularly for sensing pH, or humidity.

The hybrid composites in accordance with the present invention may also cover a wide range of colors, from metallic golden shining to pink and purple.

To further illustrate the invention, the following **examples** are provided. These examples are provided with no intend to limit the scope of the invention.

### Preparation of Starting Materials.

**Commercially available materials.** Whey protein isolate (WPI895) was purchased from Fonterra (Palmerston North, New Zealand), containing ∼70% β-lactoglobulin (BLG), ∼20% α-laCtoglobulin and ∼5% bovine serum albumin. This was purified further to ∼95% β-lactoglobulin by dialysis. Hydrogen tetrachloroaurate(III) trihydrate (HAuCl₄·3H₂O, ACS, 99.99% metal basis) was purchased from ABCR Swiss AG (Zug, Switzerland) with 49.5% Au basis. Sodium borohydride (NaBH₄) and poly(ethylene glycol) BioUltra 35,000 g mol⁻¹ (PEG) were purchased from Sigma Aldrich (now Merck KGaA, Darmstadt, Germany). HCl from AnalR NORMAPUR was obtained from VWR International (Vienna, Austria). Absolute ethanol (> 99.8%) and sodium chloride (> 99.5%) were purchased from Fischer scientific (Loughborough, UK). Aminated PS Latex (PS-NH₂) at 10% w/v and a particle diameter of 520 nm was purchased from MagSphere Inc. (California, USA). The reported crosslinking level was nil and the particles were stabilized with a cationic surfactant.

**Preparation of amyloid fibrils.** 20 g of purified BLG was dissolved in 1 L Milli-Q water (2% w/v) and adjusted to pH 2 with HCl. This was incubated at 90°C for 5 hours in an oil bath under stirring at 300 rpm. Fibrils were stored at 4°C. The BLG fibril solution was concentrated to 5% w/v by reverse osmosis against a solution of poly(ethylene glycol) 35,000 g mol⁻¹ at 100 g L⁻¹ at pH = 2.

**Preparation of amyloid fibrils-Au crystals.** 1.05 g of HAuCl₄·3H₂O salt was added into 1 L of 0.67% w/v BLG fibrils solution at pH 2 (2.6 mM HAuCl₄), the mixture was incubated for 16 hours at 60°C. Bright field imaging of the crystals was done with a 10x objective on a Zeiss AxioScope A1 microscope (Feldbach, Switzerland). Using cycles of centrifugation (30 min, 2500 g, swinging bucket rotor ref. 12870, centrifuge MPW-380R MPW Med. Instruments, Warsaw, Poland) and resuspension the solution was upconcentrated to 300 mg mL⁻¹ Au (∼1 mL) and 0.7 % w/v or 7 mg mL⁻¹ BLG fibrils.

**Preparation of amyloid fibrils-Au Nanoparticles (AuNPs).** 6 mL of 0.09 M HAuCl4 stock solution in Milli-Q water (0.35 mmol) was added to 200 mL 0.2% w/v BLG fibril dispersion followed by mixing. 3 mL of 0.074 M NaBH₄ in Milli-Q water (0.15 mmol) was added as 6 subsequent additions of 500 µL to ensure rapid mixing. The HAuCl₄:NaBH₄ ratio was 26:11 with a gold concentration of 0.5 mg mL⁻¹ and AuNPs of diameter 26 nm. This solution was up-concentrated to ∼1% w/v fibrils and ∼2.5 mg mL⁻¹ AuNPs using reverse osmosis as described above for pure BLG fibrils.

### Step (a), Fabrication of organic-inorganic hydrogels.

The 10% w/v PS-NH₂ solution was upconcentrated to 50% w/v by centrifugation (30 min, 10,000 g, fixed rotor ref. 11762).

Example (#1): Hydrogel preparation for the 1.7 g/cm3 18 karat gold crystal sample: 500 µL 300 mg mL⁻¹ Au-BLG solution (150 mg Au, 3.5 mg BLG) was mixed with 87 µL 50% PS-NH₂ solution pH =3 (39 mg PS-NH₂) and 75 µL 5% w/v BLG fibrils solution at pH = 2 (3.8 mg BLG). This results in a solution of 7.6% v/v solids, which was upconcentrated further to 15% v/v using airflow under shaking. The solution was placed in a tube with 9 mm diameter and covered with a 6-8 kDa dialysis membrane. This setup was placed in a bath of 450 mM NaCl pH = 2 to let the salt diffusion in and screen the positive charges of both the polystyrene nanoparticles and BLG fibrils to facilitate hydrogel formation.

Example (#2) For the 1.2 g/cm³ 17 karat gold crystal sample: 250 µL 300 mg mL⁻¹ Au-BLG solution (75 mg Au, 1.8 mg BLG) was mixed with 40 µL 50% PS-NH₂ solution pH=3 (20 mg PS-NH₂) and 100 µL 2% w/v BLG fibrils solution at pH=2 (2.0 mg BLG) was used. This results in a solution of 6.5% v/v solids. Hydrogel formation was done with 300 mM NaCl pH=7.

Examples (#3, 4) For the 0.7 g/cm3 (no vacuum during annealing, #4 in the table) and 0.8 g/cm3 19 karat (#3 in the table) gold crystal samples: 340 µL 300 mg mL⁻¹ Au-BLG solution (102 mg Au, 1.8 mg BLG) was mixed with 87 µL 30% PS-NH₂ solution pH =3 (26 mg PS-NH₂) and 240 µL 2% w/v BLG fibrils solution at pH = 2 (4.8 mg BLG) was used. This results in a solution of 5.2% v/v solids. Hydrogel formation was done with 300 mM NaCl pH = 2.

Example (#5) For a purple 15 karat 0.4 g/cm3 alloy: 200 µL 300 mg mL⁻¹ Au-BLG solution (60 mg Au crystals, 1.4 mg BLG) was mixed with 80 µL 44% PS-NH₂ pH 3 solution (35 mg PS-NH₂) and 6 mL 1% BLG-AuNPs (purple AuNPs as obtained with HAuCl4 and the reducing agent NaBH4) pH 2 solution. This results in a solution of 0.6% v/v solids, which was upconcentrated further to 1.2% v/v using airflow under shaking. The sample contains Au crystals which provide the majority of the gold weight. Hydrogel formation was done with 450 mM NaCl pH=2.

Comparative Example (#6): For PS-NH₂ - BLG sample: 167 µL 30% PS-NH₂ solution pH =3 (50 mg PS-NH₂) and 167 µL 2% w/v BLG fibrils solution at pH = 2 (3.3 mg BLG) was used. This results in a solution of 15% v/v solids. Hydrogel formation was done with 300 mM NaCl pH = 2. This example confirms suitability of the method and the effect of the scCO₂ drying on the PS.

Comparative Example (#7): For PS-NH₂ - BLG sample: 195 µL 44% PS-NH₂ solution pH =3 (86 mg PS-NH₂) and 300 µL 5% w/v BLG fibrils solution at pH = 2 (15 mg BLG) with 228 µL Milli-Q pH =2 was used. This results in a solution of 13% v/v solids. Hydrogel formation was done with 450 mM NaCl pH = 2.

### Step (b) Fabrication of organic-inorganic aerogel.

After diffusion of salt for 24 hours the gel was placed in an aluminum cage directly in the salt bath for 1 hour to ensure all fibril entanglement points are converted into crosslinks. The cage with the hydrogel was then transferred into a 100 mL 50% EtOH and 50% pH 2 milli-Q water bath for 24 hours. This was followed by two subsequent transfers into 100 mL 99% EtOH for 24 hours to complete the solvent exchange. Supercritical CO₂ drying was then used to remove all solvent with the aerogel as a result. The supercritical drying process was described in Nyström et al. (Adv. Mater. 2015, 28 (3), 472-478.) using CO₂ from a dip tube cylinder, a cryostat (minichiller, Huber, Offenburg, Germany), piston pump (PP200, Thar Design Inc., Pittsburg, PA, USA), temperature control (CC230, Huber) and back pressure regulator (Swagelok Niederrohrdorf, Switzerland). In summary, the hydrogels were placed in the high-pressure cell (Premex, Switzerland) with 100 mL pure ethanol. Initially, the chamber was cooled to 10°C and pressurized to 100 bar, where ethanol and CO₂ are completely miscible. The feed flow of CO₂ was then set to ∼0.019 kg min⁻¹ and the temperature was raised to 40°C. Five stasis cycles were used to ensure complete CO₂ exchange. Finally, the system was depressurized at ∼2 bar min⁻¹.

### Step (c) Fabrication of composite material.

To obtain the composite material, annealing of the aerogel was done in a vacuum oven (SalvisLab, Rotkreuz, Switzerland) for one hour at 190°C and 30 mbar. Samples were placed in the oven at room temperature and the oven reached 190°C with a heating rate of 20°C min⁻¹. Annealing parameters may vary over a broad range and readily determined by the skilled person. Particularly, annealing times may be reduced if the oven is preheated or heats up faster.

### Characterization of the materials.

The materials described herein were extensively analyzed, key properties are provided in table 1 below and the figures.

**Table 1. Summary of light gold material properties and production parameters.**

| example** | #1 | #2 | #3 | #4 | #5 |
|---|---|---|---|---|---|
| % v/v solution | 15 | 6.5 | 5.2 | 5.2 | 1.2 |
| Apparent density *ρₐₚₚ*(g/cm³) | 1.7 | 1.2 | 0.8 | 0.72* | 0.4 |
| Max. density *ρₘₐₓ* (g cm⁻³) | 3.9 | 3.9 | 3.9 | 3.9 | 2.2 |
| Porosity *Φ* (%) | 57 | 69 | 79 | 81 | 82 |
| Gold TGA (Karat) | 18 | 17 | 19 | 19 | 15 |
| T_{g}: DSC (°C) | 104 | 104 | 105 | 104 | 105 |
| Young's modulus (kPa) | 49 000 | 12 260 | 3 500 | 604 | n.d. |

| | | | | | |
|---|---|---|---|---|---|
| * no vac. ; ** all samples show golden shining, except for #5 which is purple Photographs of the 15 karat purple hybrid aerogel sample consisting of Au crystals and nanoparticles, BLG fibrils and PS-NH2. Sample of 48 mg: (A) after supercritical CO2 drying of the hydrogel and (B) after annealing of the polystyrene: app=0.4 g cm-3 and with max=2.2 g cm-3 gives a porosity of 82%. TGA: sample with 69%-3% w/w gold (∼15 karat); c.f. fig.9. | | | | | |

**Gravimetric analysis** was used to determine the density and porosity. The weight was determined using a balance and careful measurement of the aerogel volume. The apparent density was determined by dividing the mass of the solid by the geometric volume and the porosity (*Φ*) was calculated via trivial volumetric considerations using apparent and maximum density.

**Scanning electron microscopy** (SEM). The microstructure of the Au-PS sample shown in Figure 3, as well as samples with a final density of 0.7 - 1.2 g/cm3, were analyzed using scanning electron microscopy (SEM) and micrographs are shown in Figure 4. The BLG gel network (see Figure 4(D)) was shown to homogeneously encapsulate both polystyrene and the gold platelets (Figure 4(A-D)). When annealing was performed under a vacuum of 30 mbar the polystyrene particles of 520 nm merged to form a homogeneous template (Figure 4(A-C)). Annealing under atmospheric pressure resulted in partial annealing of the polystyrene particles and a significant number of individual particles can be observed in fig.4(D).

**Dynamic scanning calorimetry** (DSC) was performed using a Mettler Toledo DSC 1 STRAR^{e} System, under N₂ purging at 30 mL min⁻¹ and at 10°C min⁻¹ in perforated 40 µL aluminium crucibles. The glass transition temperature (T_{G}) was determined during the cooling cycle that followed a heating cycle as shown in Figure 5(C,D). PS-NH2 was confirmed to have a Tg at 100°C, while the PS-NH2 - BLG and light gold composite materials had a Tg of ∼105°C ; c.f. table 1. The results suggest that annealing (step c) and further processing (e.g. molding) of the inventive composite materials should be performed above 105°C.

**Thermogravimetric analysis** (TGA) combined with scanning differential thermal analysis (SDTA) was performed on a TGA/DSC3+ (Mettler Toledo) and Netzsch Jupiter STGA 449C under air atmosphere (method gas: 40 mL min-1 air, Mettler cell gas: 20 mL min-1 N₂, Netzsch protective gas 10 mL min-1 N2) by placing 5-10 mg of sample in 150 µL Mettler or 8x4x22.5 mm alumina crucibles. The temperature was increased from room temperature to 120 °C at 10°C min⁻¹ and kept for 60 min to remove all physisorbed water. The sample was then heated to 900°C at 10°C min⁻¹.

Results are provided in figure 5(A, B). For both, individual dried BLG fibrils and annealed PS-NH2, the samples completely degraded under oxidative conditions with 0% of the mass left at 680 - 700°C when using a heating rate of 10°C min-1 (Figure 5(A)). This is in line with what was previously reported. For the annealed PS-NH2 - BLG sample there was ~3% of the mass left at these final temperatures. This is expected to be NaCl that was used for the charge screening in the diffusion setup. The inventive composite materials were confirmed to be 17 - 19 karat gold, ranging from 71% w/w (74% final mass - 3% NaCl) to 80% w/w (83% final mass - 3% NaCl) gold as shown in Figure 5(B) and reported in Table 1. The inventive composites were stable up to 300°C under oxidative conditions.

**Mechanical properties** were analyzed using a Z010 Universal (Zwick GmbH & Co., Ulm, Germany) operating in compression mode using a 100 N load cell, 160 mm rod and 10 mm plate. The compression rate was 10% of the initial sample height per min (0.2 - 0.3 mm min⁻¹ for 2 - 3 mm thick samples) and measurements started at a force of 0.2 N. Compressive stress - strain curves were obtained, and the Young's modulus was determined based on the slope. Rectangular or square shaped samples were cut from the aerogels for compression analysis.

Figure 6(A) shows the compressive stress strain curves for the samples with a density of 0.7, 0.8, 1.2, 1.7 g/cm3 (bottom to top curves). Figure 3(B) shows the Young's or elastic modulus (E) that was determined from the slope from 5 - 10% compressive strain. There was a significant increase in the stiffness with increasing aerogel density (ρ_app), with the highest Young's modulus measured being 49 MPa at 1.7 g/cm3. The increase follows the general scaling behavior for samples that were annealed under vacuum, where E ∼ ρ^{α} with an exponent α of 3.5 was found. A scaling exponent of ∼3.6 is typical for porous dried gels with large mass fractal dimension. Applying of vacuum during annealing compared to atmospheric conditions had again the most significant effect with an order of magnitude higher Young's modulus for the sample annealed under vacuum with ρₐₚₚ = 0.80 g/cm3 (E = 3500) compared to the sample annealed under atmospheric pressure with ρₐₚₚ = 0.72 g/cm3 (E = 604), while prepared with identical starting solutions; c.f. Table 1.

The density of the inventive composite materials in absence of pores is 3.9 g/cm³ would, according to the rule of mixtures, lead to a Young's modulus of ~14,000 MPa. This is based on the modulus of PS (75% v/v) and BLG (5% v/v) which is for both 3000 MPa, and a Young's modulus of gold of 79,000 MPa (15% v/v). With a porosity of 57% that leads to an estimated Young's modulus of ~6000 MPa. The presence of fractal aggregates and potentially incomplete annealing of the polystyrene matrix at this material density can explain the significantly lower modulus that was found herein. However, up to 40% compression the materials did not break and returned to their original shape. For the sample with the highest density and Young's modulus (ρ_app = 1.7 g/cm³, E = 49 MPa), the Vickers Hardness was determined to be ∼10HV or 100 MPa. These results showed that density and mechanical properties of the inventive material can be tuned: depending on whether having the stiffness of polystyrene or a density lower than aluminum is the most important material property for the uses disclosed herein.

**Optical Properties.** We observe that the color of the inventive material can be varied to pink and purple by using BLG fibrils that are coated with gold nanoparticles to form the hydrogel.

**Zeta-potential measurements.** Zeta-potential measurements of colloidal dispersions were performed using the Zetasizer Nano ZS (Malvern Panalytical Ltd., Malvern, UK). Measurements were done in a 1 mL electrode cell with 0.1% w/v solutions.

## Claims

1. A composite material comprising elemental gold, amyloid fibrils and a polymer, **characterized in that**
• said elemental gold is present as single crystal gold platelets; and
• said elemental gold is homogenously distributed within said polymer; and
• said composite material having a density in the range of 0.7 - 3.9 g/cm³.

2. The composite material according to claim 1, wherein said gold
• is present as single crystal gold platelets, with the size up to 20 µm and the thickness only of 100 nm or less; and/or
• amounts to 10-99 weight-%, preferably 30 - 99% of the total weight of the composite material.

3. The composite material according to claim 1 or 2, wherein said polymer
• is obtained from a colloidal latex and / or
• is selected from the group of polyolefines, polyacrylates and polystyrenes, particularly polystyrenes.

4. The composite material according to any of the preceding claims, wherein said amyloid fibrils
• have high aspect ratio, preferably with ≤ 10 nm in diameter and ≥ 1µm in length, and / or
• have highly charged surfaces, preferably electrophoretic mobilities of the order of 2 µm 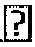 cm/V 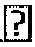 s at pH 4.

5. The composite material according to any of the preceding claims, having
• a homogeneous microstructure in which said gold platelets retain a golden shining and are embedded in the polymer matrix; and / or
• a density of preferably of 1.5 - 3.9 g/cm³, preferably of 2.5 - 3.5 g/cm³; and / or
• a T_{g} of 80 - 120 °C; and / or
• a porosity of less than 80%.

6. The composite material according to any of the preceding claims, in the form of
• a shaped article,
• a self-supporting film;
• a coating on a substrate.

7. A method for manufacturing a composite material according to any of claims 1 to 6, said method comprising the steps of :
(a) providing a first aqueous composition comprising amyloid fibrils-Gold crystals and a second aqueous composition comprising a polymer latex;
(b) combining said first and second composition followed by controlled increase of ionic strength to thereby obtain an organic-inorganic hydrogel; and
(c) converting the thus obtained hydrogel into an aerogel by removing the solvent; and then
(d) annealing the thus obtained aerogel at elevated temperatures, to thereby obtain the composite material.

8. The method according to claim 7, wherein in step (a):
• said amyloid fibrils were prepared with food-grade proteins; preferably selected from the group consisting of β-lactoglobulin, lysozyme, ovalbumin, and serum albumines; and / or
• said single crystal gold platelets are prepared by reducing an aqueous solution of gold salts which is stabilized with amyloid fibrils in colloidal state, preferably β-lactoglobulin in colloidal state.

9. The method according to any of claims 7 to 8, wherein
• in said step (b) the combined compositions are contacted with a saline solution via a diaphragm; and /or
• In said step (c) water is replaced by a low boiling organic solvent prior to solvent removal and / or scCO₂ is used to remove solvent; and / or
• In said step (d) annealing takes place at reduced pressure, preferably 10 - 100 mbar.

10. The method according to any of claims 7 to 9, additionally comprising one or more further steps (e), said further steps preferably being selected from polishing (e1), casting (e2), and coating of a substrate (e3).

11. A composite material obtainable by, or obtained according to the method of any of claims 7 to 10.

12. An article comprising or consisting of a composite material according to any of claims 1-5.

13. The article of claim 12, selected from the group consisting of
• decorative articles, which are partly or fully coated with the composite according to claim 1-5 or which are printed with an ink comprising the composite according to claim 1-5;
• ornamental articles containing of a composite material according to any of claims 1 to 5;
• electrical devices, comprising a composite material according to any of claims 1 to 5; and
• catalytic material, either in the form of monolith or in the form of granules / pellets.
